# EUROPÄISCHE PATENTANMELDUNG

(11) **EP 1 199 031 A2**
(43) Veröffentlichungstag der Anmeldung: **24.04.2002**
(21) Anmeldenummer: 01124720.2
(22) Anmeldetag: 16.10.2001
(51) Int. Cl.: A61B 5/06

(54) **Röntgenfreies intravaskuläres Lokalisierungs- und Bildgebungsverfahren**

(30) Priorität: 17.10.2000 DE 10051244
(71) Anmelder: Philips Corporate Intellectual Property GmbH, 52064 Aachen (DE); Koninklijke Philips Electronics N.V., 5621 BA Eindhoven (NL)
(72) Erfinder: Aldefeld, Bernd, Dr., 52064 Aachen (DE); Beckmann, Friedrich Karl, 52064 Aachen (DE); Eggers, Holger, 52064 Aachen (DE); Kobs, Rolf Udo Dieter, 52064 Aachen (DE); Klotz, Erhard Paul Artur, 52064 Aachen (DE); Kuhn, Michael Harald, Dr., 52064 Aachen (DE); Manke, Dirk, 52064 Aachen (DE); Rasche, Volker, Dr., 52064 Aachen (DE); Weidinger, Georg, 52064 Aachen (DE)
(74) Vertreter: Volmer, Georg, Dipl.-Ing.

(57) **Zusammenfassung**

Die Erfindung betrifft ein Verfahren und eine Vorrichtung zur Bestimmung der Position eines in ein Untersuchungsobjekt (1) eingeführten medizinischen Instruments (3) und zur Bildgebung der Umgebung des medizinischen Instruments (3). Um bei jeder Art von medizinischen Instrumenten aktuelle Positionsinformationen und Bildinformationen aus der Umgebung des medizinischen Instruments gewinnen zu können, ist erfindungsgemäß vorgesehen, dass mittels einer an dem einzuführenden Endbereich des medizinischen Instruments (3) angeordneten Lokalisierungsvorrichtung (5) die Position des medizinischen Instruments (3) in dem Untersuchungsobjekt (1) ermittelt wird, dass gleichzeitig mittels einer an dem medizinischen Instrument (3) angeordneten Bilderfassungsvorrichtung (4) Bildinformationen der Umgebung des medizinischen Instruments (3) erfasst werden und dass anhand der ermittelten Position die Position des medizinischen Instruments (3) in einem Übersichtsbild des Untersuchungsobjekts (1) angezeigt und anhand der erfassten Bildinformationen Bilder der zu der jeweiligen Position gehörigen Umgebung des Untersuchungsobjekts (1) dargestellt werden.

## Beschreibung

Die Erfindung betrifft ein Verfahren zur Bestimmung der Position eines in ein Untersuchungsobjekt eingeführten medizinischen Instruments und zur Bildgebung der Umgebung des medizinischen Instruments. Die Erfindung betrifft außerdem eine entsprechende Vorrichtung, ein entsprechendes medizinisches Instrument sowie ein Computerprogramm.

Aus der US 5,638,819 ist ein Verfahren bekannt, mit dem ein medizinisches Instrument, insbesondere eine Biopsienadel oder ein Endoskop, entlang einer gewünschten Trajektorie in einen Patienten, insbesondere in das Gehirn eines Patienten, eingerührt werden kann. Zur Lokalisierung des Instruments ist an seinem nicht in den Patienten eingeführten Teil ein Sensor angebracht, dessen Position bezüglich eines Bezugskoordinatensystems mit einer geeigneten Messeinrichtung bestimmt werden kann. Zur Navigation des Instruments wird dessen Position in ein zweidimensionales Tomographiebild eingeblendet, wobei gleichzeitig ein Live-Videobild des Endoskops angezeigt wird. Das bekannte Verfahren eignet jedoch nur zur Lokalisierung und Navigation starrer medizinischer Instrumente, da nur in diesem Fall von der Position des an dem außerhalb des Patienten befindlichen Teil des Instruments angebrachten Sensor auf die Position des in dem Patienten befindlichen Teil des Instruments geschlossen werden kann.

Der Erfindung liegt deshalb die Aufgabe zugrunde, ein Verfahren und eine Vorrichtung anzugeben, die eine Lokalisierung und Navigation beliebiger in ein Untersuchungsobjekt eingeführter medizinischer Instrumente zulassen.

Diese Aufgabe wird erfindungsgemäß durch ein Verfahren gemäß Anspruch 1 gelöst, wobei mittels einer an dem einzuführenden Endbereich des medizinischen Instruments angeordneten Lokalisierungsvorrichtung die Position des medizinischen Instruments in dem Untersuchungsobjekt ermittelt wird, wobei gleichzeitig mittels einer an dem medizinischen Instrument angeordneten Bilderfassungsvorrichtung Bildinformationen der Umgebung des medizinischen Instruments erfasst werden und wobei anhand der ermittelten Position die Position des medizinischen Instruments in einem Übersichtsbild des Untersuchungsobjekts angezeigt und anhand der erfassten Bildinformationen Bilder der zu der jeweiligen Position gehörigen Umgebung des Untersuchungsobjekts dargestellt werden.

Außerdem wird diese Aufgabe eine Vorrichtung gemäß Anspruch 10 mit
- Lokalisierungsmitteln zur Ermittlung der Position des medizinischen Instruments in dem Untersuchungsobjekt, wobei an dem einzuführenden Endbereich des medizinischen Instruments eine Lokalisierungsvorrichtung angebracht ist,
- Bildgebungsmitteln zur gleichzeitigen Erfassung von Bildinformationen der Umgebung des medizinischen Instruments, wobei an dem medizinischen Instrument eine Bilderfassungsvorrichtung angebracht ist, und
- Datenverarbeitungs- und Anzeigemitteln zur Bestimmung und Anzeige der Position des medizinischen Instruments in einem Übersichtsbild des Untersuchungsobjekts anhand der ermittelten Position und zur Bestimmung und Darstellung von Bildern der zu der jeweiligen Position gehörigen Umgebung des Untersuchungsobjekts anhand der erfassten Bildinformationen.

Der Erfindung liegt dabei der Gedanke zugrunde, dass insbesondere bei flexiblen medizinischen Instrumenten, wie beispielsweise bei Kathetern, die vor allem auch bei intravaskulären Interventionen benutzt werden, eine Lokalisierungsvorrichtung zur Bestimmung des medizinischen Instruments nicht wie bei den bekannten Verfahren an dem Teil des Instruments angebracht werden kann, der nicht in das Untersuchungsobjekt eingeführt wird. Die Anbringung optischer Sensoren oder Marker an dem aus dem Untersuchungsobjekt herausstehenden Ende eines Katheters und die Bestimmung der Position dieser Sensoren oder Marker, beispielsweise mittels eines optischen Positionsmesssystems, würde dann nämlich nicht auf die Position der in das Untersuchungsobjekt eingeführten Katheterspitze oder den Verlauf des Katheters rückschließen lassen. Erfindungsgemäß ist deshalb vorgesehen, die Lokalisierungsvorrichtung an dem Bereich des medizinischen Instruments anzuordnen, der auch in das Untersuchungsobjekt eingeführt wird, insbesondere in dem eingeführten Endbereich des medizinischen Instruments, da vor allem dessen Position während der Intervention interessiert. Um gleichzeitig auch Bildinformationen aus diesem Bereich zu gewinnen, ist erfindungsgemäß weiter vorgesehen, dort auch eine Bilderfassungsvorrichtung anzuordnen, mit der gleichzeitig zur Positionsbestimmung Bildinformationen der Umgebung des eingeführten medizinischen Instruments gewonnen werden. Die gewonnenen Bildinformationen und Positionsinformationen werden dann erfindungsgemäß dazu benutzt, die jeweils aktuelle Position des medizinischen Instruments in einem Übersichtsbild des Untersuchungsbereichs, beispielsweise bei einer intravaskulären Intervention in einem Angiogramm bzw. einer sogenannten Road-Map, anzuzeigen und gleichzeitig ein oder mehrere aktuelle Bilder des Umgebungsbereichs des medizinischen Instruments darzustellen.

Gemäß einer vorteilhaften Ausgestaltung kann das Übersichtbild aus einem drei- oder vierdimensionalen Bilddatensatz erstellt werden, wobei der vierdimensionale Bilddatensatz zusätzlich zu der Ortsauflösung auch eine zeitliche Auflösung aufweist. Das heißt, dass es zu unterschiedlichen Zeitpunkten, beispielsweise während der Herzbewegungsphase, unterschiedliche Bilddatensätze gibt, in denen vor allem unterschiedliche Positionen der untersuchten Anatomie zu den einzelnen Zeitpunkten berücksichtigt oder korrigiert sind. Die Bilddatensätze selbst können mittels eines beliebigen Bildgebungsverfahrens gewonnen sein, also beispielsweise mittels Kernspintomographie, Computertomographie, mittels eines Röntgenverfahrens, insbesondere eines dreidimensionalen Rotations-Röntgenverfahrens, oder mittels eines Ultraschallverfahrens.

Weiter kann vorgesehen sein, das Übersichtsbild bevorzugt aus einem bewegungskompensierten Bilddatensatz zu erstellen, wobei insbesondere die Herzbewegung und/oder die Atembewegung kompensiert wird, die je nach dem zu untersuchenden Bereich unterschiedliche Auswirkungen auf die Anatomie haben. Für eine derartige Kompensation sind entsprechende Verfahren bekannt. Beispielsweise kann zur Kompensation der Herzbewegung ein Elektrokardiogramm bei der Erfassung der Bilddaten erstellt werden, um die Bilddaten jeweils einer bestimmten Herzphase zuzuordnen und anhand eines Herzmodels die Auswirkungen der Herzbewegung auf die Anatomie in den einzelnen Herzbewegungsphasen zu korrigieren. Zur Korrektur der Atembewegung sind entsprechende Modelle und Sensoren bekannt, die die Atembewegung überwachen und deren Auswirkungen auf die Anatomie bestimmen und kompensieren.

In den Ansprüche 2 bis 5 sind unterschiedliche vorteilhafte Ausgestaltungen der verwendeten Lokalisierungsvorrichtung angegeben. Diese kann sowohl ein Magnetfeldsensor sein, dessen Position mittels einer externen Messvorrichtung bestimmt wird, als auch eine aktive oder passive Mikrospule, deren Position mittels einer Magnetresonanzvorrichtung bestimmbar ist, als auch ein Ultraschallsensor, der mittels einer Ultraschallvorrichtung detektierbar ist. Außerdem kann auch das medizinische Instrument wenigstens teilweise aus einem mittels einer Ultraschallvorrichtung oder einer Magnetresonanzvorrichtung detektierbaren Material bestehen. Denkbar ist auch jede andere Möglichkeit einer punktförmigen, an dem medizinischen Instrument anbringbaren Signalquelle, deren Signal von einer externen Detektorvorrichtung detektierbar ist und woraus sich die Position der Signalquelle bestimmen lässt.

Vorteilhafte Weiterbildungen für die Verwendung als Bilderfassungsvorrichtung sind in den Ansprüchen 6 bis 9 angegeben. Allen Bilderfassungsvorrichtungen gemeinsam ist, dass sie bevorzugt im einzuführenden Endbereich des medizinischen Instruments angeordnet sind, da bevorzugt Bildinformationen aus diesem Umgebungsbereich interessant sind. Die Datenübertragung der erfassten Bilddaten kann dabei drahtlos oder drahtbehaftet erfolgen. Manche Weiterbildungen der Bilderfassungsvorrichtung benötigen zum Erfassen von Bildinformationen zusätzlich noch externe Bilderfassungsmittel. Beispielsweise benötigt die MR-Vorrichtung, die bevorzugt in Form einer Mikrospule realisiert ist, in einer Ausgestaltung eine externe Anregungsspule, wie sie in einer Kernspintomographieeinrichtung verwendet wird. Andere Weiterbildungen, wie beispielsweise eine interne Anregungs- und Messspule, ein Endoskop oder eine intravaskuläre Ultraschallvorrichtung, benötigen keine zusätzlichen externen Bilderfassungsmittel.

Zur Navigation und ständigen Kontrolle der Position des medizinischen Instruments während einer Intervention wird die Lokalisierung und Bildgebung bevorzugt kontinuierlich oder in regelmäßigen Zeitabständen durchgeführt, und es werden jeweils die aktuelle Position und die zugehörigen Bilder angezeigt.

Besonders vorteilhaft wird die Erfindung zur Navigation in Blutgefäßen wie beispielsweise den Herzkranzgefäßen bei einer intravaskulären Intervention angewendet. Die Erfindung kann jedoch auch zur Navigation in anderen Kavitäten eines Untersuchungsobjekts, wie beispielsweise im Darm oder der Speiseröhre eines Patienten, eingesetzt werden.

Besonders vorteilhaft wird die Erfindung in Verbindung mit flexiblen medizinischen Instrumenten, wie beispielsweise Kathetern, verwendet, wie sie insbesondere auch bei der Untersuchung und Behandlung von Herzkranzgefäßen eingesetzt werden. Durch die Erfindung hat der Arzt beim Schieben des Katheters in dem Patienten sowohl aktuelle Informationen über die Position des Katheters als auch Bildinformationen aus dem Endbereich des Katheters zur Verfügung, um mit deren Hilfe mit hoher Genauigkeit navigieren und den Zielort finden zu können.

Die Erfindung betrifft auch eine entsprechende Vorrichtung, wie sie in Anspruch 10 angegeben ist. Weiter betrifft die Erfindung auch ein medizinisches Instrument zur Einführung in ein Untersuchungsobjekt mit einer am einzuführenden Endbereich angeordneten Lokalisierungsvorrichtung zur Ermittlung der Position des medizinischen Instruments in dem Untersuchungsobjekt und mit einer Bilderfassungsvorrichtung zur gleichzeitigen Erfassung von Bildinformationen der Umgebung des medizinischen Instruments, wobei die ermittelte Position zur Bestimmung und Anzeige der Position des medizinischen Instruments in einem Übersichtsbild des Untersuchungsobjekts und die ermittelten Bildinformationen zur Erstellung und Anzeige von Bildern der zu der jeweiligen Position gehörigen Umgebung des Untersuchungsobjekts vorgesehen sind, wie in Anspruch 11 angegeben ist. Sowohl die Vorrichtung als auch das medizinische Instrument können dabei erfindungsgemäß weitergebildet sein und vorteilhafte Ausgestaltungen aufweisen, die identisch oder analog zu den oben in Bezug auf das Verfahren ausgeführten Ausgestaltungen sind.

Die Erfindung betrifft außerdem auch ein Computerprogramm mit Programmabschnitten zur Ausführung des erfindungsgemäßen Verfahrens und/oder zur Steuerung der erfindungsgemäßen Vorrichtung bzw. des erfindungsgemäßen medizinischen Instruments, wenn das Computerprogramm auf einem Computer läuft.

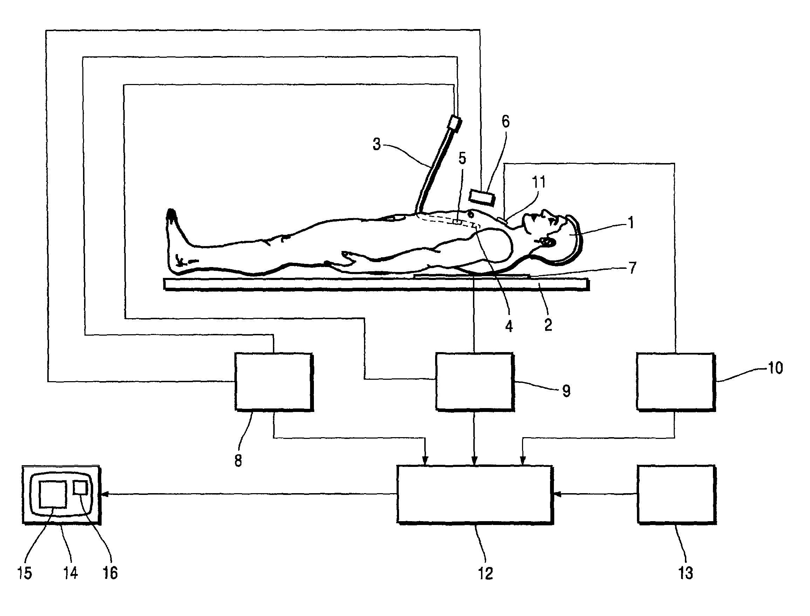

Die Erfindung wird nachfolgend anhand der einzigen Figur näher erläutert. Darin sind in einem Blockschaltbild die wesentlichen Elemente einer erfindungsgemäßen Vorrichtung gezeigt. Bei dieser Vorrichtung ist ein Patient 1 für eine intravaskuläre Intervention auf einem Patiententisch 2 liegend angeordnet. Ein erfindungsgemäßer Katheter 3 ist für eine Behandlung der Coronararterien in eine Hauptarterie des Patienten 1 eingeführt und wird dabei von einem Arzt bis zu den Herzkranzgefäßen vorgeschoben. Dieser Katheter weist an seinem in den Patienten 1 eingeführten Endbereich eine Bilderfassungsvorrichtung 4 und eine Lokalisierungsvorrichtung 5 auf. In der gezeigten Ausführungsform sei die Bilderfassungsvorrichtung 4 eine Mikrospule 4, die nach Anregung durch eine externe Anregungsspule 6 Magnetresonanz (MR)-Signale aus ihrer Umgebung empfangen kann, die Bildinformationen über die Umgebung der Mikrospule 4 liefern. Dies ist ebenso wie eine Magnetfeldspule zur Erzeugung eines statischen Magnetfeldes (nicht gezeigt) per se aus dem Bereich der Kernspintomographie bekannt und soll hier nicht weiter erläutert werden.
Weiter ist im Endbereich des Katheters 3 eine Lokalisierungsvorrichtung 5 angeordnet, die im gezeigten Ausführungsbeispiels als Magnetfeldsensor ausgebildet sei, der mit einem unterhalb des Patienten 1 angeordneten Spulentableau 7 zusammenwirkt. Mittels der von einzelnen Spulen des Spulentableaus 7 ausgesandten Signale kann anhand der von dem Magnetfeldsensor 5 empfangenen Signale die Position des Magnetfeldsensors und damit die Position des Endbereichs des Katheters 3 bestimmt werden. Auch dieses Verfahren zur Positionsbestimmung ist per se bekannt und soll hier nicht weiter erläutert werden.

Zur Verarbeitung der von der Mikrospule 4 empfangenen Signale bzw. zur Steuerung der Mikrospule 4 und der Anregungsspule 6 ist eine Bildverarbeitungs- und Steuereinrichtung 8 vorgesehen, die die gemessenen Signale in Bildinformationen umwandelt und an eine Datenverarbeitungseinrichtung 12 weiterleitet. Zur Verarbeitung der von dem Magnetfeldsensor 5 erfassten Signale und zur Steuerung des Magnetfeldsensors und des Spulentableaus 7 ist eine Positionsverarbeitungs- und Steuerungseinheit 9 vorgesehen, die die gemessenen Signale in Positionsdaten umwandelt und an die Datenverarbeitungseinrichtung 12 weiterleitet.

Um die gemessenen Positions- und Bilddaten hinsichtlich der Herzbewegung zu korrigieren, ist außerdem eine Elektrokardiographieeinrichtung 10 mit einem oder mehreren entsprechenden Sensoren 11 vorgesehen, die parallel zur Positions- und Bilddatenerfassung ein Elektrokardiogramm erfasst. Diese Daten werden ebenfalls an die Datenverarbeitungseinrichtung 12 weitergeleitet.

In der Datenverarbeitungseinrichtung 12 wird nun anhand der zugeleiteten Daten die Position des Magnetfeldsensors 5 und damit des Endbereichs des Katheters 3 bezüglich eines vorhandenen Bilddatensatzes des Untersuchungsbereichs des Patienten 1 bestimmt. Diese Bilddaten, die unmittelbar vor der Intervention oder bei einer früheren Diagnose erstellt wurden, sind in einer Datenbank 13 abgelegt und der Datenverarbeitungseinrichtung 12 zugänglich. Um eine aktuelle Position des Magnetsensors 5 in eine Position bezüglich eines aus der Datenbank 13 entnommenen Übersichtsbildes bestimmen zu können, ist außerdem eine geeignete Registrierung erforderlich, beispielsweise mittels geeigneter Marker an dem Patienten, die auch in den Übersichtsbildern enthalten sind und unmittelbar vor der Intervention als Referenzpunkte markiert werden. Im gezeigten Ausführungsbeispiel der intravaskulären Intervention wird als Übersichtsbild bevorzugt ein dreidimensionales Angiogramm, eine sogenannte Road-Map, der Herzkranzgefäße verwendet. Die Datenverarbeitungseinrichtung 12 liefert schließlich die Bildinformationen an eine Anzeigevorrichtung, beispielsweise einen Monitor, 14, auf dem einerseits das Übersichtsbild 15 angezeigt wird, in dem auch die aktuelle Position des Magnetfeldsensors 5 bzw. der Spitze des Katheters 3 eingeblendet wird. Außerdem wird daneben das aktuelle, aus den von der Mikrospule 4 erfassten Daten ermittelte Bild der Umgebung der Katheterspitze dargestellt, was als zusätzliche Information für den Arzt dienen kann, um zu bestimmen, wo sich die Katheterspitze aktuell befindet, und um besser zu navigieren. Dabei ist zur Gewinnung von Bildinformationen keine Röntgeneinrichtung erforderlich, insbesondere keine Fluoroskopieeinrichtung, die den Patienten dauerhaft während der Information mit Röntgenstrahlung belasten würde.

Das gezeigte Ausführungsbeispiel der erfindungsgemäßen Vorrichtung zeigt nur eine Variante einer solchen Vorrichtung und ist anhand konkreter Beispiele für die verwendeten Mittel erläutert worden. Insbesondere für die Art, Ausgestaltung und Anordnung der verwendeten Mittel sind zahlreiche weitere Varianten denkbar, von denen einige nachfolgend näher erläutert werden sollen.

Zusätzlich können Sensoren zur Messung der Atem- und/oder Herzbewegung an dem Patienten 1 angebracht sein, um Bewegungen der Anatomie während der Lokalisierung und Bildgebung ausgleichen zu können.

Das Übersichtsbild kann aus einem räumlich aufgelösten dreidimensionalen oder einem räumlich und zeitlich aufgelösten vierdimensionalen Bilddatensatz mit ausreichender räumlicher Auflösung erstellt sein, wobei der Bilddatensatz bei der Intervention bereits verfügbar ist. Zur Erstellung eines solchen Bilddatensatzes bieten sich verschiedene Modalitäten an. Insbesondere MRI (Magnetic Resonance Imaging), CT (Computertomographie), 3D-RX (3D Rotational X-Ray) und Ultraschalltechnik sind insbesondere für die Erfassung von Bilddaten der Coronararterien und zur Erstellung von hochaufgelösten Angiogrammen die vielversprechendsten Techniken.

Während einer Intervention muss es außerdem möglich sein, die lokale Anatomie und vor allem die Gefäßwand an der Spitze des Katheters nahezu in Echtzeit abzubilden bzw. Bilddaten davon zu liefern. Auch für bestimmte Probleme wie beispielsweise ein Steckenbleiben des Katheters oder die Auswahl des geeigneten Weges bei Verzweigungen der Arterien ist die Möglichkeit, innerhalb der Arterie nach vorne, d. h. in Bewegungsrichtung des Katheters zu schauen, sehr wichtig. Um diese Vorgaben zu erfüllen, kommen folgende Möglichkeiten in Betracht:
a) Intravaskulärer Ultraschall (IVUS) stellt derzeit zweidimensionale Ultraschallsensoren zur Verfügung, die in radialer Richtung um die Längsachse des Katheters Bildinformationen liefern können. Es sind jedoch auch dreidimensionale Ultraschallsensoren denkbar, die Bildinformationen aus allen drei Raumrichtungen liefern können.
b) Bei der optischen Kohärenztomographie (OCT) können Bilddaten bei sehr hoher räumlicher Auslösung gewonnen werden. Bei dieser Technik, die mit einfachen Optiken an der Spitze des Katheters arbeitet, können auch nicht-orthogonale Blickwinkel realisiert werden.
c) Bei der intravaskulären Magnetresonanz-Bildgebung (IVMRI) werden am Katheter eine oder mehrere Empfangsspulen in Form von Mikrospulen angebracht, wobei die Hochfrequenzanregung wie in der Figur gezeigt mittels einer Anregungsspule von außen oder von einer innen liegenden Anregungsspule erfolgt. Jedoch ist dabei auch ein starker Magnet zur Erzeugung eines starken Grundmagnetfeldes erforderlich, der den Zugang zu dem Patienten begrenzt und die Vorrichtung sehr teuer macht.
d) Auch Endoskope können als Bilderfassungsmittel eingesetzt werden, die zwar keine Bilddaten der Gefäßwand liefern, jedoch einen Blick in Richtung nach vorne ermöglichen.

Auch als Lokalisierungsvorrichtung kommen verschiedene Möglichkeiten in Betracht, von denen einige bevorzugte Varianten nachfolgend angegeben werden sollen:
a) Magnetfeldsensoren sind vor allem vielversprechend, da sie bereits miniaturisiert im Einsatz sind und eine hohe Genauigkeit der Positionsbestimmung ermöglichen.
b) Auch die Verwendung aktiver oder passiver Mikrospulen, die MR-basiert arbeiten, können zur Positionsbestimmung eingesetzt werden.
c) Auch ein Ultraschallsensor kann als Lokalisierungsvorrichtung an dem Katheter angebracht sein, wobei der Ultraschallsensor mittels eines Katheter System Interfaces (CSI) in den Tastkopf eines Ultraschallscanners integriert ist. Wenn der Ultraschallsensor in dem Katheter von dem Ultraschallscanner abgebildet wird, injiziert das Interface (CSI) einen hellen Pfeil entsprechend seiner Position. Da dies nur die Position des Katheters relativ zu der Ultraschallanordnung ergibt, muss zusätzlich auch noch die Position der Ultraschallanordnung bestimmt werden, um die ermittelten Positionen in Positionen bezüglich eines Koordinatensystems der vorliegenden Bilddaten zu übertragen. Auch eine punktförmige Ultraschallquelle kann an dem medizinischen Instrument angebracht sein, deren Signale von einem externen Sensor detektiert werden.
d) Auch auf spezielle Weise ummantelte Katheter, die mittels Ultraschall sichtbar werden, können eingesetzt werden. Damit kann auch erreicht werden, dass der gesamte Katheter anstelle nur der Katheterspitze lokalisiert und in einem Übersichtsbild angezeigt werden kann. Auch ein passives Suszeptibilitätskatheter kann verwendet werden.

Zur Visualisierung während der Intervention muss die gesamte Information auf geeignete Weise dargestellt werden, Anstelle eines Standard TV-Gerätes oder Monitors können auch beispielsweise am Kopf montierte Displays, z. B. in Form einer speziellen Brille, verwendet werden, die dem Arzt den Eindruck vermitteln, als würde er in den Patienten hineinblicken. Auch verschiedene Anzeigemodi und Betrachtungswinkel sollten möglich sein. Bevorzugt werden die aus dem Inneren der Anatomie gewonnenen Bilddaten online und quasi als Video dargestellt. Es ist jedoch auch möglich, nur Standbilder in regelmäßigen Zeitabständen und/oder mehrere Bilder aus unterschiedlichen Blickwinkeln zu generieren und dazustellen.

Außerdem sollten auch geeignete Schnittstellen zur einfachen Bedienung vorhanden sein, um einfaches Umschalten zwischen verschiedenen Modalitäten und Bilddarstellungsmodi zu ermöglichen. Auch eine Steuerung mittels Sprache oder durch Gesten kommt in Betracht. Zusätzlich kann für Notfälle auch vorgesehen sein, dass ein einfaches Röntgensystem oder eine andere bildgebende Einrichtung bereitsteht.

Mit der Erfindung ist es somit möglich, während einer Intervention ständig die aktuelle Position des medizinischen Instruments zu bestimmen und Bildinformationen aus der Umgebung des medizinischen Instruments zu gewinnen. Die Position wird außerdem in ein vorab gewonnenes Übersichtsbild eingeblendet, um eine Navigation des medizinischen Instruments zu erleichtern.
Die konkrete Ausgestaltung der verwendeten Mittel hängt insbesondere von der Art der gewünschten Intervention und der Art des Untersuchungsbereichs ab. Die Erfindung ist dabei nicht nur für flexible medizinische Instrumente wie beispielsweise Katheter geeignet, sondern grundsätzlich für jedes in einen Patienten einführbare Instrument, dessen Position von außen mit hoher Genauigkeit bestimmt werden soll.

## Patentansprüche

1. Verfahren zur Bestimmung der Position eines in ein Untersuchungsobjekt (1) eingeführten medizinischen Instruments (3) und zur Bildgebung der Umgebung des medizinischen Instruments (3), wobei mittels einer an dem einzuführenden Endbereich des medizinischen Instruments (3) angeordneten Lokalisierungsvorrichtung (5) die Position des medizinischen Instruments (3) in dem Untersuchungsobjekt (1) ermittelt wird, wobei gleichzeitig mittels einer an dem medizinischen Instrument (3) angeordneten Bilderfassungsvorrichtung (4) Bildinformationen der Umgebung des medizinischen Instruments (3) erfasst werden und wobei anhand der ermittelten Position die Position des medizinischen Instruments (3) in einem Übersichtsbild (15) des Untersuchungsobjekts (1) angezeigt und anhand der erfassten Bildinformationen Bilder der zu der jeweiligen Position gehörigen Umgebung des Untersuchungsobjekts (1) dargestellt werden.

2. Verfahren nach Anspruch 1,
**dadurch gekennzeichnet,**
**dass** als Lokalisierungsvorrichtung (5) mindestens ein Magnetfeldsensor verwendet wird, dessen Position mittels einer externen Messvorrichtung bestimmt wird.

3. Verfahren nach Anspruch 1,
**dadurch gekennzeichnet,**
**dass** als Lokalisierungsvorrichtung (5) mindestens eine aktive oder passive Mikrospule verwendet wird, deren Position mittels einer Magnetresonanzvorrichtung bestimmt wird.

4. Verfahren nach Anspruch 1,
**dadurch gekennzeichnet,**
**dass** als Lokalisierungsvorrichtung (5) ein Ultraschallsensor verwendet wird.

5. Verfahren nach Anspruch 1,
**dadurch gekennzeichnet,**
**dass** das medizinische Instrument (3) wenigstens teilweise aus einem mittels einer Ultraschallvorrichtung oder einer Magnetresonanzvorrichtung detektierbaren Material besteht.

6. Verfahren nach Anspruch 1,
**dadurch gekennzeichnet,**
**dass** als Bilderfassungsvorrichtung (4) eine Ultraschallvorrichtung, insbesondere eine intravaskuläre Ultraschallvorrichtung verwendet wird.

7. Verfahren nach Anspruch 1,
**dadurch gekennzeichnet,**
**dass** als Bilderfassungsvorrichtung (4) eine optische Kohärenztomographievorrichtung verwendet wird.

8. Verfahren nach Anspruch 1,
**dadurch gekennzeichnet,**
**dass** als Bilderfassungsvorrichtung (4) eine MR-Vorrichtung, insbesondere eine intravaskuläre MR-Vorrichtung verwendet wird.

9. Verfahren nach Anspruch 1,
**dadurch gekennzeichnet,**
**dass** als Bilderfassungsvorrichtung (4) ein Endoskop verwendet wird.

10. Vorrichtung zur Bestimmung der Position eines in ein Untersuchungsobjekt (1) eingeführten medizinischen Instruments (3) und zur Bildgebung der Umgebung des medizinischen Instruments (3) mit
- Lokalisierungsmitteln (7) zur Ermittlung der Position des medizinischen Instruments (3) in dem Untersuchungsobjekt (1), wobei an dem einzuführenden Endbereich des medizinischen Instruments eine Lokalisierungsvorrichtung (5) angebracht ist,
- Bildgebungsmitteln (6) zur gleichzeitigen Erfassung von Bildinformationen der Umgebung des medizinischen Instruments (3), wobei an dem medizinischen Instrument (3) eine Bilderfassungsvorrichtung (4) angebracht ist, und
- Datenverarbeitungs- und Anzeigemitteln (12, 14) zur Bestimmung und Anzeige der Position des medizinischen Instruments (3) in einem Übersichtsbild (15) des Untersuchungsobjekts (1) anhand der ermittelten Position und zur Bestimmung und Darstellung von Bildern der zu der jeweiligen Position gehörigen Umgebung des Untersuchungsobjekts (1) anhand der erfassten Bildinformationen.

11. Medizinisches Instrument (3) zur Einführung in ein Untersuchungsobjekt (1) mit einer am einzuführenden Endbereich angeordneten Lokalisierungsvorrichtung (5) zur Ermittlung der Position des medizinischen Instruments (3) in dem Untersuchungsobjekt (1) und mit einer Bilderfassungsvorrichtung (4) zur gleichzeitigen Erfassung von Bildinformationen der Umgebung des medizinischen Instruments (3), wobei die ermittelte Position zur Bestimmung und Anzeige der Position des medizinischen Instruments (3) in einem Übersichtsbild (15) des Untersuchungsobjekts (1) und die ermittelten Bildinformationen zur Erstellung und Anzeige von Bildern der zu der jeweiligen Position gehörigen Umgebung des Untersuchungsobjekts (1) vorgesehen sind.

12. Medizinisches Instrument nach Anspruch 11,
**dadurch gekennzeichnet,**
**dass** das medizinische Instrument (3) ein flexibles Instrument, insbesondere ein Katheter, ist.

13. Computerprogramm mit Programmabschnitten zur Ausführung des Verfahrens nach Anspruch 1 und/oder zur Steuerung einer Vorrichtung nach Anspruch 10, und/oder eines medizinischen Instruments nach Anspruch 11, wenn das Computerprogramm auf einem Computer läuft.
